(19) **Europäisches Patentamt / European Patent Office / Office européen des brevets**

(11) **EP 1 546 345 B1**

(12) # EUROPEAN PATENT SPECIFICATION

(45) Date of publication and mention
of the grant of the patent:
**28.03.2007 Bulletin 2007/13**

(51) Int Cl.:
*C12N 15/66* (2006.01)    *C12N 15/10* (2006.01)
*C12Q 1/68* (2006.01)

(21) Application number: **03793900.6**

(22) Date of filing: **05.09.2003**

(86) International application number:
**PCT/GB2003/003866**

(87) International publication number:
**WO 2004/022758 (18.03.2004 Gazette 2004/12)**

(54) **GENOME PARTITIONING**

GENOMTEILUNG

PARTITIONNEMENT DE GENOME

(84) Designated Contracting States:
**AT BE BG CH CY CZ DE DK EE ES FI FR GB GR
HU IE IT LI LU MC NL PT RO SE SI SK TR**

(30) Priority: **05.09.2002 GB 0220649
06.09.2002 GB 0220773**

(43) Date of publication of application:
**29.06.2005 Bulletin 2005/26**

(73) Proprietor: **Plant Bioscience Limited
Norwich,
Norfolk NR4 7UH (GB)**

(72) Inventor: **ZHU, Jiahui
Norwich,
Norfolk NR4 7UH (GB)**

(74) Representative: **Kremer, Simon Mark et al
Mewburn Ellis LLP
York House,
23 Kingsway
London WC2B 6HP (GB)**

(56) References cited:
EP-A- 1 001 037        WO-A-00/40757
WO-A-01/00816         WO-A-95/00530
WO-A-95/11995         WO-A-98/51789

• ALTSHULER D ET AL: "An SNP map of the human genome generated by reduced representation shotgun sequencing" NATURE, MACMILLAN JOURNALS LTD. LONDON, GB, vol. 407, 28 September 2000 (2000-09-28), pages 513-516, XP002955779 ISSN: 0028-0836 cited in the application

SEQUENCE LISTING

```
<110> Plant Bioscience Limited
Zhu, Jiahui

<120> Genome Partitioning

<130> SMK6177539

<140> PCT/GB2003/003866
<141> 2003-09-05

<150> GB 0220649.8
<151> 2002-09-05

<150> GB 0220773.6
<151> 2002-09-06

<160> 21

<170> Patent In version 3.1

<210> 1
<211> 19
<212> DNA
<213> Artificial sequence

<220>
<223> Example core sequence

<400> 1
cgtagacgat gcgtgagac          19

<210> 2
<211> 11
<212> DNA
<213> Artificial sequence

<220>
<223> Core sequence

<400> 2
ttggcgttta c          11

<210> 3
<211> 11
<212> DNA
<213> Artificial sequence

<220>
<223> Enhancing linker
```

<400> 3

cggtaaacgc c          11

<210> 4
<211> 13
<212> DNA
<213> Artificial sequence -

<220>
<223> Enhancing linker

<400> 4

aattgtaaac gcc          13

<210> 5
<211> 11
<212> DNA
<213> Artificial sequence

<220>
<223> Enhancing linker

<400> 5

atgtaaacgc c          11

<210> 6
<211> 13
<212> DNA
<213> Artificial sequence

<220>
<223> Enhancing linker

<400> 6

cuyggtaaac gcc          13

<210> 7
<211> 13
<212> DNA
<213> Artificial sequence

<220>
<223> Enhancing linker

<400> 7

gyucgtaaac gcc          13

<210> 8
<211> 15
<212> DNA
<213> Artificial sequence

<220>
<223> Enhancing linker

<400> 8

ttggcgttta cugcy          15

<210> 9
<211> 11
<212> DNA
<213> Artificial sequence

<220>
<223> Enhancing linker

<400> 9
tagtaaacgc c          11

<210> 10
<211> 13
<212> DNA
<213> Artificial sequence

<220>
<223> Enhancing linker

<220>
<221> misc feature
<222> (2)..(3)
<223> n is a or g or c or t/u, or other

<400> 10
cnnggtaaac gcc          13

<210> 11
<211> 13
<212> DNA
<213> Artificial sequence

<220>
<223> Enhancing linker

<400> 11
ttggcgttta cuy          13

<210> 12
<211> 14
<212> DNA
<213> Artificial sequence

<220>
<223> Enhancing linker

<220>
<221> misc feature
<222> (3)..(3)
<223> n is a or g or c or t/u, or other

<400> 12
ccngggtaaa cgcc          14

<210> 13
<211> 13
<212> DNA
<213> Artificial sequence

<220>
<223> Enhancing linker

<400> 13
gatcgtaaac gcc          13

<210> 14
<211> 13
<212> DNA
<213> Artificial sequence

<220>
<223> Enhancing linker

<400> 14
ggccgtaaac gcc          13

<210> 15
<211> 15
<212> DNA
<213> Artificial sequence

<220>
<223> Enhancing linker

<400> 15
ttggcgttta cgcgc          15

<210> 16
<211> 13
<212> DNA
<213> Artificial sequence

<220>
<223> Enhancing linker

<400> 16
ttggcgttta ccg          13

<210> 17
<211> 12
<212> DNA
<213> Artificial sequence

<220>
<223> Enhancing linker

<220>
<221> misc feature
<222> (2)..(2)
<223> n is a or g or c or t/u, or other

<400> 17
antgtaaacg cc          12

<210> 18
<211> 15
<212> DNA

<213> Artificial sequence

<220>
<223> Enhancing linker

<400> 18
ttggcgttta ccatg        15

<210> 19
<211> 12
<212> DNA
<213> Artificial sequence

<220>
<223> Enhancing linker

<220>
<221> misc feature
<222> (2)..(2)
<223> n is a or g or c or t/u, or other

<400> 19
gncgtaaacg cc        12

<210> 20
<211> 13
<212> DNA
<213> Artificial sequence

<220>
<223> Enhancing linker

<400> 20
tuyagtaaac gcc        13

<210> 21
<211> 13
<212> DNA
<213> Artificial sequence -

<220>
<223> Enhancing linker

<400> 21
tyuagtaaac gcc        13

**Description**

## TECHNICAL FIELD

This invention relates generally to nucleic library construction, for example for sequence variation discovery and screening. Particularly, it relates to methods and materials for reproducibly cloning a subset of a sample nucleic acid having reduced complexity.

## BACKGROUND ART

Genetic markers are of increasing importance in the genomics and proteomics fields in understanding phenotype, susceptibility to disease, and response to treatments.

Single nucleotide polymorphisms (SNPs) are one of the most abundant and useful markers, and are the subject of investigation in numerous different organisms, including within the human genome. Methods which have been used in the art have included shotgun sequencing the whole genome or sequencing PCR products (see e.g. Roth (2001) Nature Biotechnology 19: 209-211). Thus shotgun sequencing of the whole human genome provided a few millions of SNPs from five different individuals as a by-product[1] to the main initiative. A more routine method is to design a pair of specific primers for each DNA fragment of interest. After PCR amplification, the fragment can be purified and sequenced. Although these are widely used methods, their efficiency and throughput are very limited. Moreover, both of them are very costly.

Unfortunately the size of eucaryote genome make it difficult to search or screen for DNA sequence variation between individuals. To address this problem, attempts have been made to reduce the complexity of the genome to a more manageable scale, and thereby facilitate marker discovery.

AFLP is one method of achieving this. It had been widely used to study DNA polymorphisms and AFLP markers have been mapped in many species[2]. However, AFLP has not been used for SNP screening because of its technical limits, such as artificial sequence alteration, high proportion of random fragment loss and complexity of the procedure.

More recently, a more targeted and collaborative effort had been made to reduce the genome complexity for searching human SNPs.

This technology was called the reduced representation shotgun (RRS) strategy and it was adopted for the global human SNPs consortium project. RRS reduced the complexity of the genome by about six-fold, which increased the efficiency for finding the SNP. For RRS, the DNA is digested with a restriction enzyme. Based on the distribution of the fragments at different sizes, a subset of the fragments can be cut out from an electrophoresis gel so that the subset only contains the fragments with a particular size interval. The isolated fragments are subsequently be cloned into a library for random sequencing[3] (see Roth (2001) Nature Biotechnology 19: 209-211).

EP 1001037 (Whitehead Biomedical Inst., US) describes such an RRS strategy. A nucleic acid-containing sample to be assessed is treated to fractionate it into fragments selected in a sequence-dependent manner, a subset of which is selected on the basis of size.

The drawback of this method is that it can only reduce the genome complexity by a small scale.

WO 01/00816 concerns a method of attaching a fragment of a first nucleic acid molecule to a second nucleic acid molecule using adapters to mediate the binding, particularly in methods of cloning. It further concerns methods of producing fragment chains with a readily readable information content, particularly comprising fragments corresponding to code, such as alphanumeric code. The methods involve inter alia cleaving the first nucleic acid molecule with a nuclease which has a cleavage site separate from its recognition site to create at least one fragment of said first nucleic acid molecule having a single stranded nucleotide region at at least one terminus of said fragment, and subsequently binding it to an adaptor molecule.

WO 98/51789 concerns a method for preparing a normalized sub-divided library of amplified cDNA fragments from the coding region of mRNA contained in a sample using reverse transcriptase and appropriate primers, followed by digestion and ligation to an adaptor.

WO 00/40757 concerns methods for identifying nucleic acids in a sample of nucleic acids in which nucleic acids are initially present in unequal amounts. The methods include partitioning the starting population of nucleic acids to form one or more subpopulations, and then identifying nucleic acids that are present in different amounts in the partitioned nucleic acid sample as compared to the starting population

Thus it can be seen that alternative methods of reproducibly reducing the complexity of nucleic acid samples to a controllable scale e.g. for marker discovery, would provide a contribution to the art.

## DISCLOSURE OF THE INVENTION

The present inventors have developed methods to reduce the complexity of a sample of nucleic acid (e.g.

genomic or cDNA library) in large, flexible and controllable scales by dividing the genome or a collection of cDNA into smaller subsets. Briefly, the method uses multiple restriction enzymes to cut the DNA into a collection of restriction fragments. Based on the unique restriction ends of the fragments, they are then divided into different groups or "layers". A layer, or a combination of layers, is then cloned at a specific restriction site such that the resulting library only contains the desired subset or partition of the total sample. This permits the reduction of e.g. a genomic library's complexity more than a thousand-fold. By treating each sample (or pooled samples) in this way, a highly consistent sub-set of corresponding fragments is generated in each case. Thus the method has particular utility for sequence variation discovery or screening through direct sequencing. Additionally it can be utilised within automated systems to provide high-throughput screening.

Thus in a first aspect there is provided a method for producing a nucleic acid library, which library contains a plurality of different nucleic acid fragments, the combination of said fragments being a representative partition of the entirety of a sample nucleic acid, the method comprising:

(i) digesting the sample nucleic acid with a plurality of different restriction enzymes to generate a plurality of different layers of fragments,
wherein each layer is a group of fragments having a unique combination of restriction ends,
and wherein the combination of layers represents the entirety of the sample nucleic acid,
(ii) optionally purifying said fragments,
(iii) selecting a desired sub-set of layers according to the unique restriction ends of said layers,
(iv) ligating said sub-set of layers into vectors adapted to receive it,
(v) transforming host cells with the vectors
(vi) culturing said host cells to provide said library containing said partition of the sample nucleic acid, and

wherein the plurality of different restriction enzymes comprises (a) one or two enzymes which are used as cloning-end-generators to create the cloning ends selected in step (iii), and (b) four-base-cutters which destroy some or most of the fragments which could otherwise be cloned into the vectors in step (iv).

Thus the method provides a reproducible method of reducing the complexity of the sample. By selection of the appropriate numbers of restriction enzymes, the type of restriction enzymes, and the sub-set of layers ligated into said vectors, a partition with at least 10, 100, or 1000-fold reduced complexity compared to the sample nucleic acid can be generated.

In preferred embodiments, the method is performed (including, optionally, purification to remove short sequences e.g. less than 100 bps) such that the sub-set of layers ligated into said vectors provides a library with fragments with a size range of 100-2000 bps.

The number of restriction enzymes, the type of restriction enzymes, and the sub-set of layers ligated into said vectors are selected in accordance with the equations set out hereinafter.

*Choice of nucleic acid sample*

Nucleic acid for use in the present invention may include cDNA, RNA and genomic DNA. It may be provided in amplified form. RNA may be provided as cDNA.

Generally speaking, for cDNA samples, the total size of the cDNA pool will be smaller than a genome. Therefore, fewer enzymes will be used and pilot tests (see below) can be used to optimise the design.

The sample may represent all or part of a particular source of origin e.g. may have been enriched.

Nucleic acids for use in the present invention may be provided isolated and/or purified from their natural environment, in substantially pure or homogeneous form, or free or substantially free of other nucleic acids of the species of origin. Where used herein, the term "isolated" encompasses all of these possibilities.

*Choice of restriction enzymes*

In preferred embodiments, between 3 and 6 restriction enzymes will be used e.g. equal to, or at least, 3, 4, 5 or 6.

Preferably, the restriction enzymes are selected from four-, six- or eight- base-cutters.

Preferably, one or two six-base-cutters (which cut relatively rarely) are used as cloning-end-generators to create the cloning ends for the layer(s) which are selected for cloning. The other restriction enzymes are four-base-cutters (which cut relatively more frequently) and which are used, in effect, as fragment-cutters to destroy some or most of the fragments which could otherwise be cloned into the chosen vector. These enzymes,therefore serve to reduce the size of the selected layer(s). A combination of four- and six-base cutters as fragment cutters may be useful to 'hone' the size of the partition.

Preferred restriction enzymes are selected from any of those given in Table 1. Eight-base cutters include SfiI and NotI. More preferably the enzymes HpaII, AluI, DraI, and PstI are used (PstI being used to generate cloning ends).

However those skilled in the art will appreciate that other combinations of enzymes may be selected as appropriate to the specific application in hand - for instances when all or part of a reference sequence for a sample is known, the enzymes will be selected such as to have a target frequency appropriate to the size of the partition which it is wished to generate. Likewise if it is desired to investigate a particular region of the sample, the enzymes will be selected such as to achieve this.

Preferably the plurality of enzymes are used simultaneously, and are selected such as to be active under comparable conditions to permit this. Optimum conditions for commercially available restriction enzyme are available from the manufacturers.

Restriction by one enzyme may be partial. In such cases it is preferred that the group of fragments in the selected layer have restriction ends created by said partial digestion.

*Choice of layers*

In preferred embodiments, the selected sub-set of layers consists of one layer or two layers

The following represent various preferred embodiments of the invention:

*Design of partitions for samples with unknown sequence and size*

In some embodiments it may be required to generate a partition having a desired number of unique fragments where no reference sequence is available in a genome of unknown size. In this case the present invention may incorporate the performance of a 'pilot test' to confirm the validity of the partition design, and optionally to refine it.

A pilot test may be used to measure the size or complexity (number of unique sequences) of a particular partition design. It will also provide information about original genome size and restriction site frequencies. The principle is as follows: when sequencing a library (e.g. a partition) having a given number of colonies, there will be a chance for a particular sequence to be sequenced more than once. This is called sequence redundancy of shotgun sequencing strategy. The more colonies sequenced the more redundancy. The smaller (or less complex) the library, the more redundancy. Thus assessment of sequence redundancy provides information about the size of the partition.

The function is described in this formula:

$$F = n(n-1)/\sum_{i} n_i(n_i - 1) \pm s \ .$$

Wherein:

F is the size or complexity of the partition
n is the total number of good sequences obtained by sequencing
ni is the number of sequence in the ith contig.
s is the standard error, which represents the statistical error when the sample size is not big enough.

Thus, for example, 500 colonies may be selected from a partition and sequenced. This should give more than 400 good quality sequences. Using these sequences, the complexity of the partition, F, can be calculated. Additionally, the deviation constant for restriction enzymes in the genome can be extrapolated from the sequence results permitting a honing of the partition design.

Thus the method may include performing the method of the invention as described above using parameters which are likely to produce an acceptable result for a wide spread of genome sizes from different species, for example by performing a digestion of 5μg genomic DNA using a 6nt cutter (e.g. PstI) as the cloning site enzyme and three 4nt cutters (e.g. HpaII, AluI and DraI). The partition may be cloned into pZErO at PstI site with presence of suitable enhancing linkers (linkers for HpaII, AluI and DraI).

The following steps are then performed:

(vii) sequencing the fragments in a fraction of the colonies (host cells) in said library,
(viii) calculating the size of the library (i.e. partition) using formula $F = n(n\text{-}1)/\Sigma_i\, n_i(n_i\text{-}1) \pm s.$

If the partition size is appropriate it can be accepted.

If not (for example it is too small or too big) then the following further steps, in any appropriate order, may be performed:

(ix) providing the restriction site frequency ($f_i$) of the enzymes used in the partition, for example based on sequences obtained at step(vii),

(x) calculating the genome size G using the formula:

$$N_{x1 \sim x2} = GP_1^2 \sum_{k=x1}^{k=x2} \prod_{i=1}^{i} (1 - P_i)^k$$

wherein:

$N_{x1 \sim x2}$ is the number of fragments with length between x1 and x2 (which is F above).
$k$ is fragment length
x1 and x2 are upper and lower limits of the size range of the fragments in the library (these may be assumed as 100bp and 2000bp, as described above, or can be verified by the sequence obtained)
$P_i$ is the probability of having a restriction site at any given base for the 'i' th enzyme,

(xi) providing a restriction site frequency ($f_i$) for enzymes not used in the partition, for example based on sequences obtained at step(vii) (this can also be expressed as $P_i$),

(xii) selecting further restriction enzymes on the basis of restriction site frequency ($f_i$) to generate a desired size of partition using the formula:

$$N_{x1 \sim x2} = GP_1^2 \sum_{k=x1}^{k=x2} \prod_{i=1}^{i} (1 - P_i)^k$$

(xiii) producing a further nucleic library in accordance with steps (i)-(vi) using at least one of these further restriction enzymes.

It should be noted that in reality the possibility of an enzyme cutting site being present will vary according to the restriction enzyme in question. Preferably, where a sample sequence is unknown, therefore $P_i$ is measured or estimated in silico based on a large number sample of sequences e.g. from a database.

A corresponding approach may be used with cDNA from an unknown tissue from an unknown species. In such case the lower complexity (compared with a genome) suggests that PstI as the cloning site restriction enzyme, and HpaII as the fragment cutter, may be an appropriate starting point.

*Design of partitions for samples of known size and unknown sequence*

Where the approximate genome size (G) is known, in choosing the enzymes to be used in step (i), the restriction site frequency may be assumed to be randomly distributed i.e. the v = 1, wherein, v is the deviation constant in the formula P=v/256 for four base cutter and P=v/1096 for six base cutter.

The enzymes to produce a desired partition size are thus selected on the basis of the formula:

$$N_{x1 \sim x2} = GP_1^2 \sum_{k=x1}^{k=x2} \prod_{i=1}^{i} (1 - P_i)^k \; ,$$

More specifically the formula:

$$N' = 4^{-12} v' G \sum_{k=x1}^{k=x2} \left[ (1-1/4^4)^{nk} (1-1/4^6)^{(1+m)k} \right]$$

wherein:

$k$ is fragment length (and x1 and x2 are upper and lower limits)
G is the size of the genome
n is the number of extra 4 nt cutters
m is the number of extra 6 nt cutters

is used to select an appropriate combination of 4nt and 6nt cutters.

This can be verified as described above in steps (vii)-(xiii) if required.

A corresponding approach may be used with cDNA from tissues or species in which the complexity is known or can be estimated, either directly or by comparison with other species.

*Samples with known sequence*

One or more reference sequences corresponding to the sample nucleic acid may be known. It will be understood that the sample nucleic acid sequence (inasmuch as it derives from a different source from the reference) is likely to include sequence variation with respect to any reference and indeed this variation between corresponding sequences underlies certain embodiments of the present invention. Nevertheless, since such variations are by definition rare, the reference sequence can be used to calculate restriction site frequency for restriction enzymes which it may be desired to use in the methods described herein.

When the sequence is known, the restriction site frequency of each enzyme can be provided, and the formula:

$$N_{x1 \sim x2} = GP_1^2 \sum_{k=x1}^{k=x2} \prod_{i=1}^{i} (1-P_i)^k$$

can be used to select the enzymes to produce a desired partition size,

Where a reference sequence is known, a set of restriction enzyme can be based on the restriction map of the desired genes and other sequences so as to select them in particular, while still having an appropriately sized partition.

Some particular practical aspects of the invention will now be discussed in more detail:

*Purification*

In preferred embodiments the fragments are purified at step (ii).

As described in the Examples hereinafter, fragments may be purified in a conventional manner. In examples herein, the restriction reaction was passed through a column containing resins (QIAQuick PCR purification kit, QiaGen), which can effectively adsorb DNA molecules larger than 100bp. After washing with 70% ethanol, the DNA fragments were eluted into 30~50$\mu$l water. An alternative second method used the BioRad Clean-A-Gene kit. The third method was to purify the fragments by running 1% agarose gel and recovering the DNA by using Promega gel recovery kit. For the third method, extra DNA should be used, for example, 10 microgram for rice and pearl millet, 20 microgram for human and wheat.

Preferred purification techniques will be such as to remove fragments of less than 100 bases.

*Enrichment of sample*

Where a corresponding reference sequence is known, an enrichment strategy may be adopted, so that a particular region or gene may be treated. For example, when a particular set of fragments are required to be enclosed, restriction enzymes may be chosen through a restriction map of the reference sequence(s). Moreover, if a particular set of genes are needed to be studied, from the reference sequence, a set of oligos (16-60 bases preferably 20-50 bases) could be designed to enrich the genes e.g. via a hybridization method using magnetic beads with biotin-labelled oligonucleotides attached on them (see e.g. Edwards KJ, Barker JHA, Daly A, Jones C, Karp A (1996) Microsatellite libraries enriched for several microsatellite sequences in plants. BioTechniques 20:758-760). This technique may be particularly useful when dealing with repetitive DNA.

Once the sample is enriched, it may be preferred to use pilot tests to confirm the size of the total DNA pool.

*Enhancement linkers*

In preferred embodiments, enhancement linkers are added prior or during step (iv) such that only the desired sub-set of layers being included in said library. The linkers prevent fragments with compatible restriction ends combining to form artifacts.

Such linkers (which may be provided as a pair of oligonucleotides) comprise:

(i) a core sequence, which is selected such that it does not contain a restriction site and does not have a high probability of hybridizing to target sequence,
(ii) a portion that matches the appropriate restricted-end
(iii) additional sequence to prevent the linkers annealing e.g. an overhang.

The enhancement linkers are not used for the cloning site restriction enzyme(s).

Preferred linkers are any of those given in Table 1.

*Cloning and ligation*

The terms "cloning" and "ligation" and so on are used herein because they will be well understood by those skilled in the art, and can be performed by standard techniques. Those skilled in the art are well able to cloned selected fragments into libraries - see, for example, *Molecular Cloning: a Laboratory Manual:* 2nd edition, Sambrook *et al,* 1989, Cold Spring Harbor Laboratory Press or *Current Protocols in Molecular Biology*, Second Edition, Ausubel et al. eds., John Wiley & Sons, 1992 (or later editions of these works) both of which are specifically incorporated herein by reference. Generally speaking a typical protocol can be achieved by exposing a vector restricted with the appropriate enzymes to the selected layers such as to ligate or otherwise incorporate the heterologous nucleic acid fragments into the vector at the appropriate cloning site; exposing the ligation product (recombinant vector) to host cells under conditions whereby the vector is taken up by the cells such as to generate a population of host cells containing the vector; exposing the population of cells to a propagation medium comprising a selection agent whereby transformed host cells which contain vector incorporating the nucleic acid insert are selectively grown or propagated in the medium.

Where desired, one or more pairs of "adaptor" oligonucleotides may be used to bridge the cloning ends of the DNA fragments of interest (i.e. from the layer(s) in the desired sub-set) and the cloning site of the vector(s). The adaptor sequences have appropriate restriction site sequences (fragment and vector) at each end and a core sequence in the middle. An example core sequence is 5-CGTAGACGATGCGTGAGAC-3.

In such cases, PCR amplification may optionally be used to enrich the fragments of interest and increase the amount of DNA by using the adaptor sequence as PCR primer. This may be advantageous where the quantity of fragments is relatively low.

Thus, prior to step (iv), the method may optionally include the step of ligating adaptor oligonucleotides to all or part (e.g. generally one or both layers, if two layers are selected) of the selected sub-set of fragments in order to facilitate their ligation into vectors adapted to receive them.

The adaptor sequences may optionally incorporate extra restriction sites.

*Use for discovery of sequence variation*

As described in more detail below, the sample may comprise corresponding nucleic acid from several (e.g. two or more) different sources. This permits equivalent partitions to be compared e.g. for the discovery of sequence variation.

The methods described herein may be used to identify any type of marker e.g. microsatellites, minisatellites etc. Preferably the markers are SNPs.

The size of the partition sequences will be chosen to be appropriate to the number and nature of markers which it is desired to look for. Thus, for example, if 'S' different SNPs are required, it may be appropriate to ensure that there are at least that many different unique sequences in the partition (more preferably twice that many) representing a total length of S x 1000 bases.

Markers can be investigated which are appropriate to the samples. For example, the nucleic acid-containing sample can be pooled from individuals who share a particular trait (e.g. an undesirable trait, such as a particular disorder, or a desirable trait, such as resistance to a particular disorder). Sequences can be taken from different species, varieties or populations such as to provide markers for plant-breeding, or phylogenetic studies etc. Preferred target genomes (or cDNA sources) include Human, Arabidopsis, wheat, rice, millet and soybean genomes.

Thus the invention provides a method for identifying a limited population of markers in a sample nucleic acid, which method comprises:

(a) providing sample nucleic acid from at least 2 different sources,
(b) providing a representative partition of the sample nucleic acid in accordance with the methods described herein,
(c) identifying differences within corresponding sequences from said different sources contained within the library.

The nucleic acid from different sources may be pooled. However it may also be analysed on separate occasions since the methods of the invention produce a partition of fixed size and fixed content in a reproducible manner.

Generally the corresponding sequences from the different sources within the partition are sequenced to identify the differences. Such sequence data is obtained by sequencing the library e.g. to 3 -5 times coverage. If desired the actual size of partition can be calculated as described herein.

The term "corresponding to" in terms of sequence comparisons herein (whether with a known reference, or between different source nucleic acids in a sample) refers to sequences derived from equivalent loci or genes from two different genomes (e.g. the sequences may be orthologues, homologues, alleles etc.) but which may therefore include differences between them (e.g. by way of mutation, polymorphism, or other sequence variation which gives rise to nucleic acid "markers").

Corresponding sequences will generally be at least 80% identical, most preferably at least about 90%, 95%, 96%, 97%, 98% or 99% identical. Identity is established by comparison of the full length of the sequences (or the shorter of the sequences). Thus alignment of different sequencing results, and assessment of the degree of identity between them, can be used to confirm that sequences are indeed corresponding ones, and hence that sequence differences between them represent potential markers. For markers which are candidate single nucleotide polymorphisms, the frequency should preferably not exceed 1% of the total number of bases in the shorter of the two sequences - sequences which meet these criteria may be selected as corresponding. Whether sequences are indeed corresponding sequences showing intergenomic or inter-gene variation, rather than e.g. multiple copies in a single genome or individual, can be verified if desired by conventional methods familiar to those skilled in the art of SNP identification. For example, intergenome or inter-gene-copy variation is generally larger than the allelic variation so that a phylogenetic tree of the sequences in an alignment based on sequence similarity may distinguish the two types of variation. If required, SNP candidates can be validated by genotyping and genetic mapping - if the marker segregates and can be mapped to a chromosomal location, it would normally be recognized as true allelic variation.

*Use in genotyping*

Many uses of SNPs require: (i) the SNP's map position in the human genome, and (ii) a genotyping assay for scoring the locus in association studies.

Methods for assessment of polymorphisms are reviewed by Schafer and Hawkins, (Nature Biotechnology (1998)16, 33-39, and references referred to therein) and include: allele specific oligonucleotide probing, amplification using PCR, denaturing gradient gel electrophoresis, RNase cleavage, chemical cleavage of mismatch, T4 endonuclease VII cleavage, multiphoton detection, cleavase fragment length polymorphism, E.coli mismatch repair enzymes, denaturing high performance liquid chromatography, (MALDI-TOF) mass spectrometry, analysing the melting characteristics for double stranded DNA fragments as described by Akey et al (2001) Biotechniques 30; 358-367.

The assessment of polymorphisms may be carried out on a DNA microchip. One example of such a microchip system may involve the synthesis of microarrays of oligonucleotides on a glass support. Fluorescently - labelled PCR products may then be hybridised to the oligonucleotide array and sequence specific hybridisation may be detected by scanning confocal microscopy and analysed automatically (see Marshall & Hodgson (1998) Nature Biotechnology 16: 27-31, for a review).

Thus the invention also provides for a method for making a genotyping microchip for use in assaying a limited population of polymorphisms within a sample (see, e.g., U.S. Pat. Nos. 5,861,242 and 5,837,832).

As with other reduced representation approaches, the present invention can facilitate efficient genotyping. Once a set of polymorphisms is isolated, probes or primers for detecting those polymorphisms can be incorporated into such a chip. When it is desirable to assay an individual for the polymorphisms in the set, nucleic acid is isolated from that individual, and it can be partitioned with the same methods that were used to isolate the original set of polymorphisms.

However, this invention is more flexible than the other reduced representation approaches because it can greatly and flexibly reduce the size of a partition e.g. to as small as one containing 500 unique fragments.

For example, if one wishes to genotype a new sample for 10,000, or 1000 or 100 SNPs isolated from a specific partition, one could restriction-digest the sample; isolate an appropriate partition; and amplify by PCR using primers complementary to a generic linker. The resulting amplification products could be hybridized to an appropriate 'genotyping array'. Such methods allow the user to concentrate study on only a limited portion of the entire spectrum of the available polymorphisms. By examining only a limited portion of the genome, this method has the added benefit of reducing cross-reactivity between unrelated genetic sites.

*Use for investigation of methylation sensitivity*

For methylation sensitivity studies, methylation sensitive and non-sensitive restriction enzymes may be used separately so that the methylation distribution patterns could be revealed by comparing the two.

*Computer-implemented embodiments*

In a further aspect of the present invention, some or all of the steps of the methods described above may be performed by a digital computer, in particular steps in designing appropriate genome partitions based on reference sequence restriction maps and\or equations as described above. Although this could be done using commercially available sequence analysis software and sequence databases, in preferred embodiments a bespoke system directly provides the choice of enzymes to use.

Thus the invention provides an automated computer system, comprising a combination of hardware and software, that can rapidly determine optimised partitions based on a reference sequence, a desired size, and optionally desired region within the sequence.

Preferably, these aspects of the invention are implemented in computer programs executing on a programmable computer comprising a processor, a data storage system (including volatile and nonvolatile memory and/or storage elements), at least one input device, and at least one output device. Data input through one or more input devices for temporary or permanent storage in the data storage system includes sequences. Program code is applied to the input data to perform the functions described above and generate output information. The output information is applied to one or more output devices, in known fashion.

The program code will include analysis of some or all of the functions described above, and will include the ability to input a reference sequence, and preferences regarding partition size and optionally preferred regions to include in the partition. The program code will also be able to reference (e.g. from a look-up table) restriction site target sequences for different 4 and 6nt cutters.

The automated system can be implemented through a variety of combinations of computer hardware and software. In one implementation, the computer hardware is a high-speed multiprocessor computer running a well-known operating system, such as UNIX. In other embodiments personal computers using single or multiple microprocessors might also function within the parameters of the present invention.

Each such computer program is preferably stored on a storage media or device (e.g., ROM or magnetic diskette) readable by a general or special purpose programmable computer, for configuring and operating the computer when the storage media or device is read by the computer to perform the procedures described herein. The inventive system may also be considered to be implemented as a computer-readable storage medium, configured with a computer program, where the storage medium so configured causes a computer to operate in a specific and predefined manner to perform the functions described herein.

The invention will now be further described with reference to the following non-limiting Figures and Examples. Other embodiments of the invention will occur to those skilled in the art in the light of these.

Example 1 - methods for determining size of layers and partitions

*Relationship between Enzymes and Layers*

When DNA is digested with more than one restriction enzymes, the DNA fragments can be classified into groups based on the restriction ends produced specifically by the restriction enzymes.

When N different enzymes are used, the maximum number of groups of DNA fragments generated, which are called "layers" herein, is:

$$L = N + (N^2 - N)/2$$

Each layer of DNA fragments can be specifically cloned into a cloning vector at the corresponding restriction site. The specificity is determined by the cloning site, which only matches the restriction fragment ends of the chosen layers.

*Combinations of Layers*

In principle, any combination of the layers can be cloned into a library. The sub-set or combination of layers cloned is termed a "partition" herein. The number of possible partitions will be:

$$P = C_L^1 + C_L^2 \cdots + C_L^{L-1}.$$

For example, when five different enzymes were used, there should be up to 15 layers and 32766 partitions. In practice, it is preferred to use only a partition containing one or two layers for library construction. Given that more than a hundred of restriction enzymes are available on the market, the number of possible partition of a genome is huge.

*Estimating number and size of fragments per layer*

The size of a layer depends on the number and the types of enzymes used.
For a given cloning site generated by a 6nt cutter,

```
Total number of fragments = total number of restriction sites =
```

$$\frac{vG}{4^6}.$$

(*G* stands for genome size in base pairs).
(*v* is the frequency deviation for each particular enzyme in a particular genome, and may be assumed to be 1 unless known or established to be otherwise).

The possibility of a restriction fragment with length $\geq k$ is

$$(1 - 1/4^6)^k.$$

The possibility of obtaining a fragment with length of *k* is

$$(1 - 1/4^6)^k - (1 - 1/4^6)^{k+1}$$

The number of fragments with length between x1 and x2 is

$$N = 4^{-6}vG[(1 - 1/4^6)^{x1} - (1 - 1/4^6)^{x2}].$$

With an extra 4nt cutter, the number of fragments per layer will be reduced because a given fragment could be cut internally, to generate fragments with different combinations of restriction ends, and hence no long within the original layer. Thus the fragments per layer will be reduced to: $N' = 4^{-12}v'G\sum_{x1}^{x2}\left[(1 - 1/4^4)^k(1 - 1/4^6)^k\right].$

With two extra 4nt cutters, $N' = 4^{-12} v' G \sum_{x1}^{x2} \left[ (1 - 1/4^4)^{2k} (1 - 1/4^6)^k \right].$

With three extra 4nt cutters, $N' = 4^{-12} v' G \sum_{x1}^{x2} \left[ (1 - 1/4^4)^{3k} (1 - 1/4^6)^k \right].$

With n extra 4nt cutters, $N' = 4^{-12} v' G \sum_{x1}^{x2} \left[ (1 - 1/4^4)^{nk} (1 - 1/4^6)^k \right].$

With an extra 6nt cutter, the number of fragments will be reduced to $N' = 4^{-12} v' G \sum_{x1}^{x2} \left[ (1 - 1/4^6)^{2k} \right].$

With two extra 6nt cutters, $N' = 4^{-12} v' G \sum_{x1}^{x2} \left[ (1 - 1/4^6)^{3k} \right].$

If one 6nt cutter is used for cloning site, a 4nt extra cutter and 'm' 6nt extra cutters are used, the number of fragments will be $N' = 4^{-12} v' G \sum_{x1}^{x2} \left[ (1 - 1/4^4)^{nk} (1 - 1/4^6)^{(1+m)k} \right].$ Herein v' is a combined frequency deviation so that this formula is preferred to be used only when v' is assumed to be one or when pilot test is used to verify the partition design.

In general, the number of fragments with length between x1 and x2 (in base pairs) is

$$N_{x1\sim x2} = G P_1^2 \sum_{k=x1}^{k=x2} \prod_{i=1}^{i} (1 - P_i)^k ,$$ in which $P_i$ is the possibility to have a restriction site at any base pair for the *'i' th* enzyme used and $P_1$ represents that for the enzyme of the cloning site.

It should be noted that when a partition is based on fragments having two different restriction ends, the number of matching fragments remains the same. Although the number of total fragments is doubled with two enzymes, the chance of having two different ends is 50%. Therefore, the size of a partition with one cloning end is the same as that with combination of two different cloning ends if other restriction enzymes (fragment cutters, the enzymes which do not match the cloning site) are the same. Thus for the purposes of calculation, the two restriction enzymes for the cloning site may be counted as one enzyme, with the $P_1$ taken as the mean of that of the two enzymes.

In preferred embodiments, most cloned fragments will fall between 100 and 2000 base pairs (and hence x1 and x2 may be assumed as 100 bp and 2000 bp). This is because smaller fragments, which are not informative, may be removed by purification techniques. Additionally, the selected restriction endonuclease(s) will generally cleave the sample nucleic acid molecule at least approximately every 2000 bases. Thus larger fragments will be comparatively rare.

*Testing the number of unique fragments - "pilot testing"*

Since the frequency of a given restriction site varies greatly from enzyme to enzyme and from genome to genome, the frequency of the enzymes and the actual size of designed partitions needs to be tested unless it is known from a pre-existing sequence.

To evaluate the number of unique fragments in a partition. After the library of a partition is constructed in accordance with the above, randomly pick and sequence 500 well-separated colonies. Assemble them so that the same sequences will be piled in alignments. Each alignment of a sequence may be termed a "contig" or "clique". The number of unique fragments in the partition should be $F = n(n-1)/\Sigma_i n_i (n_i - 1) \pm s$, in which $n$ is the total number of sequence and $n_i$ is the number of the sequences in the ith contig. When the number of sequences is big enough, the standard error s could be neglected. (See Appendix I where the derivation is given)

Example 2 - Use of a partition to find DNA sequence variation

*Partition strategy*

Clearly, the larger the partition, the more sequence reactions are needed to get sequence pair-wise comparison. It is therefore preferred to keep the size of the partition to the minimum likely to encompass the number of sequence variations which it is desired to identify.

For example, when if five hundred SNPs are required for a population or a panel of varieties, the partition should provide more than five hundreds unique sequences (ideally about 1000). Random sequencing should preferably cover the library 3-5 times - more than 10-times should not be necessary.

The number and types of restriction enzymes should be decided based on the formulae described above. When the genome sequence is available, the restriction site frequency can be checked and a particular design to cover certain genomic regions or genes can be performed using a known or bespoke programs. Sequence enrichment strategy can also be considered at that stage.

For a new species and a particular set of enzymes, a pilot test is carried out to confirm the expected size of the partition is valid in respect of that genome. For cDNA, a pilot test may be required in each case to hone the partitioning.

*Sample preparation*

This can be done in conventional manner. For e.g. rice DNA, at least two microgram is preferred. For the human genome, more than five microgram DNA is recommended for normal genome partitioning without gel-based purification.

*Restriction digestion*

Restriction digestion can be performed in one cocktail. However, if the enzymes are optimal in different conditions, two or even three stages of reaction should be carried out.

Partial digestion can be used as a special way to enlarge a partition. Normally, partial digestion is only performed on one enzyme, which generates the cloning ends.

*Use of Enhancing Linkers*

For ligation, enhancing linkers can be designed to avoid chimerical sequences and restoring the undesired restriction site during ligation. In the Examples herein, each linker consists of two oligos. The core sequence were 5'-TTGGCGTTTAC-3' and 3'-CCGCAAATG-5'.

In order to define the core sequence, a set of randomly generated short sequences were Blast searched against all sequences from different species in EMBL database. 5'-GGCGTTTAC-3' was selected on the basis that it had the least hits, and it did not contain a restriction site.

One end of the linker has a overhang 'TT' so that no linkage can be made at this end. The other end has a sticky end with added nucleotides, which matches the restriction sites - this can be linked to the genomic DNA fragments with undesired restriction ends. Because of the competition of these linkers, DNA fragments with the same restriction site as the linkers will not link to each other to create "false" fragments within given layers.

Thus for each used restriction enzyme (except that for cloning site) a corresponding enhancing linker should be added into the ligation reaction. In preferred embodiments the final concentration of each oligo should be 0.1μM. This is conveniently achieved using a stock solution of each oligo (1mM) (which can be stored for use e.g. at -20°C. Before ligation, a 'cocktail' of these oligos is made to contain each necessary oligo with the concentration of 10μM and 1μl of the cocktail should be added in the 100μl ligation reaction.

Preferred enhancing linkers are listed in Table 1 hereinafter. The restriction endonuclease in the list is recommended for genome partitioning.

*Cloning*

This can be done in conventional manner. Zero Background vector from Invitrogen was used. Ligation, transformation, colonies picking, miniprep and sequencing were performed using routine DNA library construction protocols.

Compatibility with Two automated systems (Qiagen Robots 3000 and 8000 with QIAprep 96 Turbo BioRobot Kit) was demonstrated showing the utility of the invention in high-throughput screening.

Example 3 - SNP discovery in rice

Rice is a model plant for cereals. DNA sequences are widely available for rice subspecies, Indica and Japonica. The rice genome is about 400 million base pairs and has been shot-gun sequenced independently by several groups, while at least one other group (Japanese National Rice Genome Project) is using a BAC strategy. Currently, sequences from Huada[4] and RGP[5] are publicly available for Indica and Japonica respectively.

Genomic DNA was isolated from 20 rice varieties and equally pooled into one sample (Table 2 below).

Ten μg of the pooled DNA was digested with 0.5 μl of HpaII, AluI, DraI and PstI each in a cocktail with GIB buffer 8. The total volume of reaction was 100μl and it was incubated at 37 °C for 12 hours overnight.

The digested DNA was purified using QIAQuick PCR purification kit, QiaGen. The purified DNA was eluted in 20 μl water and subsequently 5μl of the purified DNA fragments were used in a 10μl ligation reaction. Six oligos (as three enhancing linkers for HpaII, AluI and DraI) were added into the reaction. They were 5'-TTGGCGTTTAC-3', 5'-CGGTAAACGCC-3', 5'-TTGGCGTTTAC-3', 5'-GTAAACGCC-3', 5'-TTGGCGTTTAC-3', 5'-AATTGTAAACGCC-3' (see Table 1). The final concentration of each oligo was 0.1μM. One μl of ligase was used and 0.2μg pZero vector (InvitroGen) digested with PstI was added. The reaction was at 15°C for 30 minutes and then kept at -20°C for subsequent transformation.

The one-shot competent cell (InvitroGen) was used for transformation of the E. coli. Kanamycin was used as selection antibiotic. After overnight culture on LB medium agar plate, approximately 600 colonies were selected. The colonies were cultured in 1.5ml LB medium and the plasmid DNA was isolated using QuiaGen miniprep kit. Thirty of the plasmid DNA samples were run on agarose gel to see the size of inserts. Out of the thirty samples, the insert size ranged from 200 to 3000 bp, with average of 800bp. The DNA was sequenced using fluorescent-capillary method on ABI 3700 (sequence service was provided by John Innes Centre).

The sequences were processed with PreGap4 to cut away the poor sequence and vector sequence. The sequence with good quality (pregap4 default threshold was used for quality control) can be assembled into contigs using Gap4.

About 400 pairwise comparisons were found (Table 3), from which 278 SNP candidates were identified.

**Table 3 Number of sequences and SNP candidates**

| No. of sequences in each contig | No. of Contig | No. of sequences in each contig type | No. of SNP candidates |
|---|---|---|---|
| 1 | 212 | 212 | - |
| 2 | 121 | 242 | 222 |
| 3 | 8 | 24 | 46 |
| 4 | 2 | 8 | 6 |
| 6 | 1 | 6 | 0 |
| 8 | 1 | 8 | 4 |
| Total | 345 | 500 | 278 |

Using the formula: $F=n(n-1)/\Sigma_i n_i(n_i-1)\pm s$, the size of the partition was estimated as containing 624 unique colonies (the standard error was ignored as being insignificant) (Table 3). In this calculation, F = 500x(500-1)/[212x1x(1-1)+121x2x(2-1)+8x3x(3-1)+2x4x(4-1)+1x6x(6-1)+1x8x(8-1)]≈624;

The average insert size of the colonies was 800bp. Since rice genome is 400 million bp and the size of library was (624 x 800)bp, the genome partition was about 1/800 of the whole genome. In another word, this genome partitioning design reduced the complexity of the library by 800 times.

Example 4 - SNP discovery in Pearl millet

Pearl millet (Table 4) was tested using the procedure set out in Example 3. The total number of sequences was 607 from about 800 colonies. The result showed that a partition containing about 2000 colonies were constructed.

Since the size of pearl millet genome is not known accurately, the actual reduction in complexity of the genome was not determined, nor has the total number of SNPs been calculated.

**Table 4 Pearl millet varieties pooled for genome partitioning experiment**

| | |
|---|---|
| 1. | Tift238D |
| 2. | IP10401 |
| 3. | IP10402 |
| 4. | IP8214 |
| 5. | 81B |
| 6. | ICMP451 |
| 7. | LGD-1 |
| 8. | ICMP85410 |
| 9. | Tift23DB |
| 10. | 843B |

(continued)

| 11. | P7 |
| 12. | PT732B |
| 13. | P1449 |
| 14. | 841B |
| 15. | 863B |
| 16. | H77 |
| 17. | PRLT2 |
| 18. | ICMP501 |
| 19. | Tift383 |
| 20. | 700481-21-8 |

References

1. J. Craig Venter, et al. 2001. Science 291:1304-1315.
2. P. Vos, et al. 1995. Nucleic Acids Res 23:4407-4414.
3. D. Altshuler, et al. 2000. Nature 407: 513-516.
4. Hua Da rice sequence database:
http://210.83.138.53/rice/tools.php
5. Japanese sequence database: http://rgp.dna.affrc.go.jp/

**Table 1 Sequences of enhancing linkers**

*Acc* I
5'-TTGGCGTTTAC-3'
5'-ATGTAAACGCC-3'
5'-CGGTAAACGCC-3'
*Aci* I
5'-TTGGCGTTTAC-3'
5'-CGGTAAACGCC-3'
*Afl* III
5'-TTGGCGTTTAC-3'
5'-CUYGGTAAACGCC-3'
*Alu* I
5'-TTGGCGTTTAC-3'
5'-GTAAACGCC-3'
*Apo* I
5'-TTGGCGTTTAC-3'
5' -AATTGTAAACGCC-3'
*Ban* I
5'-TTGGCGTTTAC-3'
5'-GYUCGTAAACGCC-3'
*Ban* II
5'-TTGGCGTTTACUGCY-3'
5'-GTAAACGCC-3'
*Bfa* I
5'-TTGGCGTTTAC-3'
5'-TAGTAAACGCC-3'
*BsaA* I
5'-TTGGCGTTTAC-3'
5'-GTAAACGCC-3'

(continued)

*BsaH* I
5'-TTGGCGTTTAC-3'
5'-CGGTAAACGCC-3'

*BsaJ* I
5'-TTGGCGTTTAC-3'
5'-CNNGGTAAACGCC-3'

*BsiE* I
5'-TTGGCGTTTACUY-3'
5'-GTAAACGCC-3'

*BssK* I
5'-TTGGCGTTTAC-3'
5'-CCNGGGTAAACGCC-3'

*BstN* I
None is needed.

*BstU* I
5'-TTGGCGTTTAC-3'
5'-GTAAACGCC-3'

*Btg* I
5'-TTGGCGTTTAC-3'
5'-CUYGGTAAACGCC-3'

*Cac8* I
5'-TTGGCGTTTAC-3'
5'-GTAAACGCC-3'

*DpnI*
5'-TTGGCGTTTAC-3'
5'-GTAAACGCC-3'

*Dpn* II
5'-TTGGCGTTTAC-3'
5'-GATCGTAAACGCC-3'

*Dra* I
5'-TTGGCGTTTAC-3'
5'-AATTGTAAACGCC-3'

*Eae* I
5'-TTGGCGTTTAC-3'
5'-GGCCGTAAACGCC-3'

*Fnu4H* I
None is needed.

*Hae* II
5'-TTGGCGTTTACGCGC-3'
5'-GTAAACGCC-3'

*Hae* III
5'-TTGGCGTTTAC-3'
5'-GTAAACGCC-3'

*Hha* I
5'-TTGGCGTTTACCG-3'
5'-GTAAACGCC-3'

*Hinc* II
5'-TTGGCGTTTAC-3'
5'-GTAAACGCC-3'

*Hinf* I
5'-TTGGCGTTTAC-3'

(continued)

5'-ANTGTAAACGCC-3'

*HinP1* I

5'-TTGGCGTTTAC-3'

5'-CGGTAAACGCC-3'

*Hpa* II

5'-TTGGCGTTTAC-3'

5'-CGGTAAACGCC-3'

*Hpy188* I

None is needed.

*HpyCH4* III

None is needed.

*HpyCH4* IV

5'-TTGGCGTTTAC-3'

5'-CGGTAAACGCC-3'

*HpyCH4* V

5'-TTGGCGTTTAC-3'

5'-GTAAACGCC-3'

*Mbo* I

5'-TTGGCGTTTAC-3'

5'-GATCGTAAACGCC-3'

*Mnl* I

None is needed.

*Mse* I

5'-TTGGCGTTTAC-3'

5'-TAGTAAACGCC-3'

*Msl* I

None is needed.

*Msp* I

5'-TTGGCGTTTAC-3'

5'-CGGTAAACGCC-3'

*Nla* III

5'-TTGGCGTTTACCATG-3'

5'-GTAAACGCC-3'

*Nla* IV

5'-TTGGCGTTTAC-3'

5'-GTAAACGCC-3'

*Nsp* I

5'-TTGGCGTTTACCATG-3'

5'-GTAAACGCC-3'

*Rsa* I

5'-TTGGCGTTTAC-3'

5'-GTAAACGCC-3'

*Sau3A* I

5'-TTGGCGTTTAC-3'

5'-GATCGTAAACGCC-3'

*Sau96* I

5'-TTGGCGTTTAC-3'

5'-GNCGTAAACGCC-3'

*ScrF* I

None is needed.

*Sfc* I

(continued)

5'-TTGGCGTTTAC-3'

5'-TUYAGTAAACGCC-3'

*Sml* I

5'-TTGGCGTTTAC-3'

5'-TYUAGTAAACGCC-3'

*Taq* I

5'-TTGGCGTTTAC-3'

5'-CGGTAAACGCC-3'

*Tsp509* I

5'-TTGGCGTTTAC-3'

5'-AATTGTAAACGCC-3'

*CviJ* I

None is needed.

*CviT* I

None is needed.

Table 2 20 Rice Varieties

| Series No. | RC No. | IRGC No. | Name |
| --- | --- | --- | --- |
| 1 | 1 | 25833 | AusJhari |
| 2 | 8 | 25885 | Lakhsnikajal |
| 3 | 10 | 25898 | Mimidim |
| 4 | 17 | 27502 | Walanga |
| 5 | 18 | 27522 | Ashmber |
| 6 | 21 | 33118 | Hnanwa |
| 7 | 26 | 34737 | Bawoi |
| 8 | 27 | 38697 | NPE837 |
| 9 | 28 | 62154 | ASU |
| 10 | 33 | 64780 | Kalshori |
| 11 | 36 | 64792 | Narikel Jhupi |
| 12 | 40 | 64887 | Dagpa Bara |
| 13 | 48 | 66513 | Guru Muthessa |
| 14 | 50 | 66529 | Podi Niyanwee |
| 15 | 58 | 66614 | Puteh Kaca |
| 16 | 81 | 67423 | Aguyod |
| 17 | 88 | 67720 | Banikat |
| 18 | 98 | 71496 | Babalatik |
| 19 | 178 | 78333 | Khau Muong Pieng |
| 20 | 181 | 78369 | Nep Ngau |

**Appendix I** Derivation of formula, $F=n(n-1)/\Sigma_i n_i(n_i-1) \pm s$. Assume a pool which has F different/unique sequences and each unique sequence has very large equal number of copies. Then the size of this pool, in terms of genome partitioning, is F. The chance to randomly selecting a pair of sequences that are the same is 1/F, because the pool is very large so that taking one sequence off the pool makes almost no difference to the size.

If P is the total number of pair wise combinations of the same sequences and P' is the total number of any pair wise combinations, the chance to randomly selecting a pair of sequences that are the same is also P/P'. Thus, F=P'/P. If n is the total number of sequences of the pool. P'=n(n-1)/2. If $n_i$ is the number of sequences of the ith unique sequence (or contigs). *i* is from 1 to F.

$$P=[n_1(n_1-1)+n_2(n_2-1)\ldots+n_F(n_F-1)]/2=\sum_1^F n_i(n_i-1)/2=\sum_i n_i(n_i-1)/2.$$

Therefore, $F=n(n-1)/\Sigma_i n_i(n_i-1)$.

If the number of sequences is small as we are sampling the pool, there will be a statistical error, which is given as S. As the result, $F=n(n-1)/\Sigma_i n_i(n_i-1)\pm s$.

## Claims

1.  A method for producing a nucleic acid library,

    which library contains a plurality of different nucleic acid fragments, the combination of said fragments being a representative partition of the entirety of a sample nucleic acid,
    the method comprising:

    (i) digesting the sample nucleic acid with a plurality of different restriction enzymes to generate a plurality of different layers of fragments,
    wherein each layer is a group of fragments having a unique combination of restriction ends,

and wherein the combination of layers represents the entirety of the sample nucleic acid,

(ii) optionally purifying said fragments,

(iii) selecting a desired sub-set of layers according to the unique restriction ends of said layers,

(iv) ligating said sub-set of layers into vectors adapted to receive it,

(v) transforming host cells with the vectors

(vi) culturing said host cells to provide said library containing said partition of the sample nucleic acid, and wherein the plurality of different restriction enzymes comprises (a) one or two enzymes which are used as cloning-end-generators to create the cloning ends selected in step (iii), and (b) four-base-cutters which destroy some or most of the fragments which could otherwise be cloned into the vectors in step (iv).

2.  A method as claimed in claim 1 wherein the sample is genomic DNA.

3.  A method as claimed in any one of the preceding claims wherein the number of and type of the different restriction enzymes used in step (i), and the sub-set of layers selected in step (iii) are selected in order to generate a library size with a reduced complexity compared to the sample nucleic acid of at least 10, 100, or 1000-fold.

4.  A method as claimed in any one of the preceding claims wherein between 3 and 6 restriction enzymes are used.

5.  A method as claimed in any one of the preceding claims wherein the digestion by one restriction enzyme is partial, and the group of fragments in the selected layer have restriction ends created by said partial digestion.

6.  A method as claimed in any one of the preceding claims wherein the selected sub-set of layers consists of one layer or two layers.

7.  A method as claimed in any one of the preceding claims wherein the fragments are purified at step (ii).

8.  A method as claimed in any one of the preceding claims wherein the size range of the fragments in the library is between 100 and 2000 bps.

9.  A method as claimed in any one of the preceding claims wherein linkers are added prior or during step (iv) to prevent undesired sub-sets of layers being included in said library,

    each of which linkers comprises:

        (i) a core sequence,
        (ii) a portion that matches the restricted-end of an undesired sub-set, and
        (iii) a sequence to inhibit the fragments in the undesired sub-set recombining.

10. A method as claimed in claim 9 wherein the linkers comprise any of those given in Table 1.

11. A method as claimed in any one of the preceding claims wherein adaptor oligonucleotides are used in step (iv) to facilitate the ligation of the desired sub-set of layers into vectors adapted to receive it.

12. A method as claimed in any one of the preceding claims wherein libraries are prepared separately using methylation sensitive and non-sensitive restriction enzymes, whereby comparison of the libraries permits methylation distribution patterns in the sample to be revealed.

13. A method as claimed in any one of the preceding claims wherein the sequence of the sample nucleic acid is known, and the number of and type of the different restriction enzymes used in step (i), and the sub-set of layers selected in step (iii) are selected to produce the desired library size in accordance with the restriction site frequency of each enzyme in the sample nucleic acid sequence, and are selected in accordance with the formula:

$$N_{x1 \sim x2} = G P_1^{2} \sum_{k=x1}^{k=x2} \prod_{i=1}^{i} (1 - P_i)^k$$

Nx1~x2 is the number of fragments with length between x1 and x2

k is fragment length
x1 and x2 are upper and lower limits of the size range of the fragments in the library
Pi is the probability of having a restriction site at any given base for the 'i'th enzyme
G is the size of the sample.

**14.** A method as claimed in any one of claims 1 to 12 wherein the size of the sample nucleic acid is known, and the number of and type of the different restriction enzymes used in step (i), and the sub-set of layers selected in step (iii) are selected to produce the desired library size in accordance with an assumed restriction site frequency of each enzyme in the sample nucleic acid, and are selected in accordance with the formula:

$$N_{x1 \sim x2} = GP_1^{\,2} \sum_{k=x1}^{k=x2} \prod_{i=1}^{i} (1 - P_i)^k$$

Nx1~x2 is the number of fragments with length between x1 and x2
k is fragment length
G is the size of the sample
x1 and x2 are upper and lower limits of the size range of the fragments in the library
Pi is the probability of having a restriction site at any given base for the 'i'th enzyme.

**15.** A method as claimed in claim 14 wherein the restriction enzymes used in step (i) are 4 and 6nt cutting restriction enzymes, and are selected on the basis of the formula:

$$N' = 4^{-12} v' G \sum_{\int k=x1}^{\int k=x2} \left[ (1 - 1/4^4)^{nk} (1 - 1/4^6)^{(1+m)k} \right]$$

wherein:

k is fragment length
G is the size of the sample
x1 and x2 are upper and lower limits of the size range of the fragments in the library
n is the number of extra 4 nt cutters
m is the number of extra 6 nt cutters.

**16.** A method as claimed in claim 14 or claim 15 wherein the size of the resulting library is estimated by the further steps of:

(vii) sequencing the fragments in a fraction of the host cells in said library,
(viii) estimating the size of the library using formula:

$$F = n(n-1) / \sum_i n_i (n_i - 1) \pm s$$

wherein:

F is the estimated size of the library
n is the total number of sequences obtained by sequencing,
ni is the number of sequence in the ith contig,
s is the standard error.

**17.** A method as claimed in claim 16 wherein an library is generated by the further steps of:

(ix) providing a restriction site frequency for enzymes not used in step (i), optionally using the sequence information obtained at step (vii),

(x) selecting further restriction enzymes on the basis of restriction site frequency to generate a desired size of partition using the formula given in claim 16,

(xi) producing an nucleic library in accordance with steps (i)-(vi) using at least one of these further restriction enzymes,

(xii) optionally repeating steps (vii) to (xi) until the desired library size is obtained.

**18.** A method as claimed in any one of claims 1 to 12 wherein the size of the sample nucleic acid is unknown, and the number of and type of the different restriction enzymes used in step (i), and the sub-set of layers selected in step (iii) are selected to produce the desired library size in accordance with an assumed restriction site frequency of each enzyme in the sample nucleic acid; and

wherein the size of the resulting library is estimated by the further steps of:.

(vii) sequencing the fragments in a fraction of the host cells in said library,

(viii) estimating the size of the library using formula:

$$ F = n(n-1) / \sum_i n_i (n_i - 1) \pm s $$

wherein:

F is the estimated size of the library

n is the total number of sequences obtained by sequencing,

ni is the number of sequence in the ith contig,

s is the standard error.

**19.** A method as claimed in claim 18 wherein three 4nt- and one 6nt- cutting restriction enzymes are used in step (i).

**20.** A method as claimed in claim 18 or claim 19 wherein the size of the sample is estimated by the further steps of:

(ix) providing the restriction site frequency of the enzymes used in step (i), optionally using the sequence information obtained at step(vii),

(x) calculating the sample size G using the formula:

$$ N_{x1\sim x2} = G P_1^2 \sum_{k=x1}^{k=x2} \prod_{i=1}^{i} (1 - P_i)^k $$

wherein:

Nx1~x2 is the number of fragments with length between x1 and x2

k is fragment length

x1 and x2 are upper and lower limits of the size range of the fragments in the library

Pi is the probability of having a restriction site at any given base for the 'i'th enzyme

G is the size of the sample.

**21.** A method as claimed in claim 20 wherein an library is generated by the further steps of:

(xi) providing a restriction site frequency for enzymes not used in step (i), optionally using the sequence information obtained at step(vii),

(xii) selecting further restriction enzymes on the basis of restriction site frequency to generate a desired size of partition using the formula given in claim 20,

(xiii) producing an nucleic library in accordance with steps (i)-(vi) using at least one of these further restriction enzymes,

(xiv) optionally repeating steps (vii) to (xiii) until the desired library size is obtained.

22. A method as claimed in any one of the preceding claims wherein the sample nucleic acid comprises nucleic acid from two or more different sources which are pooled to produce a library comprising fragments from each.

23. A method for identifying a limited population of markers in a sample nucleic acid, which method comprises:

(a) providing sample nucleic acid from at least two different sources,
(b) providing a library containing a representative partition of the sample nucleic acid in accordance with any one of claims 1 to 22,
(c) identifying differences within corresponding sequences from said different sources contained within the library.

24. A method as claimed in any one of claims 1 to 23 for producing a nucleic acid library, which library contains a plurality of different nucleic acid fragments, the combination of said fragments being a representative partition of the entirety of a sample nucleic acid, wherein the method is preceded by the steps of:

(a) providing a digital computer, which computer comprises a processor, a data storage system, at least one input device, and at least one output device,
(b) running a program code on said computer, which operates on the input of one or both of:

(I) a reference sequence or restriction map from the sample nucleic acid,
(II) a preference regarding partition size, and optionally preferred region of the sample to include in the partition,

wherein the program code performs a function in accordance with the following formula such as to provide an output of the number of and type of the different restriction enzymes to be used in step (i) of the method, and the sub-set of layers to be selected in step (iii) of the method, such as to produce a desired library size:

$$N_{x1 \sim x2} = GP_1^2 \sum_{k=x1}^{k=x2} \prod_{i=1}^{i} (1 - P_i)^k$$

Nx1~x2 is the number of fragments with length between x1 and x2 k is fragment length
x1 and x2 are upper and lower limits of the size range of the fragments in the library
Pi is the probability of having a restriction site at any given base for the 'i' th enzyme.
G is the size of the sample.

25. A computer program for selecting the number of and type of the different restriction enzymes used in step (i), and the sub-set of layers selected in step (iii) of the method of any one of claims 1 to 23, which computer program code operates on the input of one or both of:

(i) a reference sequence or restriction map from the sample nucleic acid,
(ii) a preference regarding partition size, and optionally preferred region of the sample to include in the partition,

and wherein the program code includes a look up table including reference restriction site target sequences for different 4 and 6nt cutting restriction enzymes, and wherein the program code performs a function in accordance with the following formula such as to provide an output of the number of and type of the different restriction enzymes to be used in step (i) of the method, and the sub-set of layers to be selected in step (iii) of the method, such as to produce a desired library size:

$$N_{x1 \sim x2} = GP_1^2 \sum_{k=x1}^{k=x2} \prod_{i=1}^{i} (1 - P_i)^k$$

Nx1~x2 is the number of fragments with length between x1 and x2

k is fragment length

x1 and x2 are upper and lower limits of the size range of the fragments in the library

Pi is the probability of having a restriction site at any given base for the 'i' th enzyme.

G is the size of the sample.

26. A computer program as claimed in claim 25 which is stored on a storage media or device readable by a general or special purpose programmable computer.

27. A system for selecting the number of and type of the different restriction enzymes used in step (i), and the sub-set of layers selected in step (iii) of the method of any one of claims 1 to 23, which system comprises program code run on a digital computer, which is the program code of claim 25 or claim 26, and which computer comprises a processor, a data storage system, at least one input device, and at least one output device.

**Patentansprüche**

1. Verfahren zur Herstellung einer Nucleinsäurebibliothek; wobei die Bibliothek eine Vielzahl an unterschiedlichen Nucleinsäurefragmenten umfasst, wobei die Kombination dieser Fragmente einen repräsentativen Anteil der Gesamtheit einer Proben-Nucleinsäure darstellen, wobei das Verfahren Folgendes umfasst:

(i) den Verdau der Proben-Nucleinsäure mit eine Vielzahl an verschiedenen Restriktionsenzymen, um eine Vielzahl an unterschiedlichen Sätzen oder Lagen von Fragmenten herzustellen, worin jeder Satz eine Gruppe von Fragmenten mit einer einzigartigen Kombination von Restriktionsenden darstellt, und worin die Kombination von Sätzen die gesamte Proben-Nucleinsäure darstellt,
(ii) gegebenenfalls das Reinigen der Fragmente,
(ii) das Auswählen einer gewünschten Untergruppe von Sätzen entsprechend den einzigartigen Restriktionsenden der Sätze,
(iv) das Ligieren der Untergruppe von Sätzen in Vektoren, die so beschaffen sind, dass sie diese aufnehmen können,
(v) das Transformieren von Wirtszellen mit den Vektoren,
(vi) das Kultivieren der Wirtszellen, um die Bibliothek mit dem Anteil der Proben-Nucleinsäure bereitzustellen, und

worin die Vielzahl an unterschiedlichen Restriktionsenzymen (a) ein oder zwei Enzyme, die als Klonierungsenderzeuger verwendet werden, um die in Schritt (iii) ausgewählten Klonierungsenden zu schaffen, und (b) einen 4-Basen-Spalter umfasst, der manche oder die meisten der Fragmente zerstört, die ansonsten in Schritt (iv) in die Vektoren kloniert werden könnten.

2. Verfahren nach Anspruch 1, worin die Probe genomische DNA ist.

3. Verfahren nach einem der vorangegangenen Ansprüche, worin die Anzahl und Art der in Schritt (i) verwendeten unterschiedlichen Restriktionsenzyme und die in Schritt (iii) ausgewählte Untergruppe von Sätzen so gewählt werden, dass eine Bibliothekengröße mit zumindest 10-, 100- oder 1000fach verringerter Komplexität im Vergleich zur Proben-Nucleinsäure erzeugt wird.

4. Verfahren nach einem der vorangegangenen Ansprüche, worin zwischen 3 und 6 Restriktionsenzyme verwendet werden.

5. Verfahren nach einem der vorangegangenen Ansprüche, worin der Verdau mit einem Restriktionsenzym partiell ist und die Gruppe von Fragmenten im ausgewählten Satz Restriktionsenden aufweist, die durch den partiellen Verdau geschaffen werden.

6. Verfahren nach einem der vorangegangenen Ansprüche, worin die ausgewählte Untergruppe von Sätzen aus einem Satz oder aus zwei Sätzen besteht.

7. Verfahren nach einem der vorangegangenen Ansprüche, worin die Fragmente in Schritt (ii) gereinigt werden.

8. Verfahren nach einem der vorangegangenen Ansprüche, worin der Größenbereich der Fragmente in der Bibliothek zwischen 100 und 2000 bp liegt.

9. Verfahren nach einem der vorangegangenen Ansprüche, worin vor oder während Schritt (iv) Linker hinzugefügt werden, um zu verhindern, dass unerwünschte Untergruppen von Sätzen in die Bibliothek aufgenommen werden, wobei jeder der Linker Folgendes umfasst:

   (i) eine Kernsequenz,
   (ii) einen Teil, der zum restringierten Ende einer unerwünschten Untergruppe passt, und
   (iii) eine Sequenz zur Hemmung einer Rekombination der Fragmente in der unerwünschten Untergruppe.

10. Verfahren nach Anspruch 9, worin die Linker beliebige der in Tabelle 1 angeführten umfassen.

11. Verfahren nach einem der vorangegangenen Ansprüche, worin Adapter-Oligonucleotide in Schritt (iv) eingesetzt werden, um die Ligation der gewünschten Untergruppe von Sätzen in Vektoren, die so beschaffen sind, dass sie diese aufnehmen können, zu erleichtern.

12. Verfahren nach einem der vorangegangenen Ansprüche, worin Bibliotheken unter Einsatz von gegen Methylierung empfindlichen und unempfindlichen Enzymen getrennt hergestellt werden, wodurch ein Vergleich der Bibliotheken die Entdeckung von Methylierungsverteilungsmustern in der Probe ermöglicht.

13. Verfahren nach einem der vorangegangenen Ansprüche, worin die Sequenz der Proben-Nucleinsäure bekannt ist und die Anzahl und Art der in Schritt (i) verwendeten unterschiedlichen Restriktionsenzyme und die in Schritt (iii) ausgewählte Untergruppe von Sätzen so gewählt werden, dass die gewünschte Bibliothekengröße in Übereinstimmung mit der Restriktionsstellenhäufigkeit jedes Enzyms in der Proben-Nucleinsäuresequenz erzeugt wird, und in Übereinstimmung mit der Formel

$$N_{x1 \sim x2} = G P_1^2 \sum_{k=x1}^{k=x2} \prod_{i=1}^{i} (1 - P_i)^k$$

gewählt werden,

   worin Nx1~x2 die Anzahl an Fragmenten mit einer Länge zwischen x1 und x2 ist,
   k die Fragmentlänge ist,
   x1 und x2 die Ober- und Untergrenze des Größenbereichs der Fragmente in der Bibliothek sind,
   Pi die Wahrscheinlichkeit ist, dass an einer vorgegebenen Base eine Restriktionsstelle für das ‚i'-te Enzym vorliegt, und
   G die Größe der Probe ist.

14. Verfahren nach einem der Ansprüche 1 bis 12, worin die Größe der Proben-Nucleinsäure bekannt ist und die Anzahl und Art der in Schritt (i) verwendeten unterschiedlichen Restriktionsenzyme und die in Schritt (iii) ausgewählte Untergruppe von Sätzen so gewählt werden, dass die gewünschte Bibliothekengröße in Übereinstimmung mit einer angenommenen Restriktionsstellenhäufigkeit der einzelnen Enzyme in der Proben-Nucleinsäuresequenz erzeugt wird, und in Übereinstimmung mit der Formel

$$N_{x1\sim x2} = GP_1^2 \sum_{k=x1}^{k=x2} \prod_{i=1}^{i} (1-P_i)^k$$

gewählt werden,

worin Nx1~x2 die Anzahl an Fragmenten mit einer Länge zwischen x1 und x2 ist,
k die Fragmentlänge ist,
G die Größe der Probe ist,
x1 und x2 die Ober- und Untergrenze des Größenbereichs der Fragmente in der Bibliothek sind und
Pi die Wahrscheinlichkeit ist, dass an einer vorgegebenen Base eine Restriktionsstelle für das 'i'. Enzym vorliegt.

**15.** Verfahren nach Anspruch 14, worin die in Schritt (i) verwendeten Restriktionsenzyme 4- und 6-nt-spaltende Restriktionsenzyme sind und auf Basis der Formel:

$$N' = 4^{-12} v' G \sum_{\int k=x1}^{\int k=x2} \left[ (1-1/4^4)^{nk} (1-1/4^6)^{(l+m)k} \right]$$

ausgewählt werden, worin:

k die Fragmentlänge ist,
G die Größe der Probe ist,
x1 und x2 die Ober- und Untergrenze des Größenbereichs der Fragmente in der Bibliothek sind,
n die Anzahl an zusätzlichen 4-Nucleotid-Spaltern ist,
m die Anzahl an zusätzlichen 6-Nucleotid-Spaltern ist.

**16.** Verfahren nach Anspruch 14 oder Anspruch 15, worin die Größe der resultierenden Bibliothek durch den folgenden weiteren Schritt abgeschätzt wird:

(vii) das Sequenzieren der Fragmente in einer Fraktion der Wirtszellen in der Bibliothek,
(viii) das Abschätzen der Größe der Bibliothek mithilfe der Formel:

$$F = n(n-1) / \sum_i n_i(n_i - 1) \pm s$$

worin:

F die geschätzte Größe der Bibliothek ist,
n die Gesamtanzahl an durch Sequenzierung erhaltenen Sequenzen ist,
ni die Anzahl an Sequenzen im i-ten Contig ist und
s der Standardfehler ist.

**17.** Verfahren nach Anspruch 16, worin eine Bibliothek durch die folgenden weiteren Schritte erzeugt wird:

(ix) das Bereitstellen einer Restriktionsstellenhäufigkeit für Enzyme, die nicht in Schritt (i) verwendet werden, gegebenenfalls unter Verwendung von in Schritt (vii) erhaltenen Sequenzinformationen,
(x) das Auswählen weiterer Restriktionsenzyme auf Basis der Restriktionsstellenhäufigkeit, um eine gewünschte Anteilsgröße mithilfe der in Anspruch 16 angegebenen Formel zu erzeugen,
(xi) das Herstellen einer Nucleinsäurebibliothek gemäß den Schritten (i) bis (vi) unter Einsatz zumindest eines dieser weiteren Restriktionsenzyme,
(xii) gegebenenfalls das Wiederholen der Schritte (vii) bis (xi), bis die gewünschte Bibliothekengröße erreicht ist.

**18.** Verfahren nach einem der Ansprüche 1 bis 12, worin die Größe der Proben-Nucleinsäure unbekannt ist, und die Anzahl und Art der in Schritt (i) verwendeten unterschiedlichen Restriktionsenzyme und die in Schritt (iii) ausgewählte Untergruppe von Sätzen so gewählt werden, dass die gewünschte Bibliothekengröße in Übereinstimmung mit einer angenommenen Restriktionsstellenhäufigkeit der einzelnen Enzyme in der Proben-Nucleinsäuresequenz erzeugt wird, und
worin die Größe der resultierenden Bibliothek durch die folgenden weiteren Schritte abgeschätzt wird:

> (vii) das Sequenzieren der Fragmente in einer Fraktion der Wirtszellen in der Bibliothek,
> (vii) das Abschätzen der Größe der Bibliothek mithilfe der Formel:

$$F = n(n-1)/\sum_i n_i(n_i-1) \pm s$$

> worin:

> > F die geschätzte Größe der Bibliothek ist,
> > n die Gesamtanzahl an durch Sequenzierung erhaltenen Sequenzen ist,
> > ni die Anzahl an Sequenzen im i-ten Contig ist und
> > s der Standardfehler ist.

**19.** Verfahren nach Anspruch 18, worin in Schritt (i) drei 4-nt- und ein 6-nt-spaltende Restriktionsenzyme eingesetzt werden.

**20.** Verfahren nach Anspruch 18 oder Anspruch 19, worin die Größe der Probe durch die folgenden weiteren Schritte abgeschätzt wird:

> (ix) das Breitstellen der Restriktionsstellenhäufigkeit der in Schritt (i) verwendeten Enzyme, gegebenenfalls unter Verwendung von in Schritt (vii) erhaltenen Sequenzinformationen,
> (x) das Berechnen der Probengröße G mithilfe der Formel:

$$N_{x1 \sim x2} = GP_1^2 \sum_{k=x1}^{k=x2} \prod_{i=1}^{i} (1-P_i)^k$$

> worin:

> > Nx1~x2 die Anzahl an Fragmenten mit einer Länge zwischen x1 und x2 ist,
> > k die Fragmentlänge ist,
> > x1 und x2 die Ober- und Untergrenze des Größenbereichs der Fragmente in der Bibliothek sind,
> > Pi die Wahrscheinlichkeit ist, dass an einer vorgegebenen Base eine Restriktionsstelle für das ‚i'-te Enzym vorliegt, und
> > G die Größe der Probe ist.

**21.** Verfahren nach Anspruch 20, worin eine Bibliothek durch die folgenden weiteren Schritte erzeugt wird: ,

> (xi) das Bereitstellen einer Restriktionsstellenhäufigkeit für Enzyme, die nicht in Schritt (i) verwendet werden, gegebenenfalls unter Verwendung von in Schritt (vii) erhaltenen Sequenzinformationen,
> (xii) das Auswählen weiterer Restriktionsenzyme auf Basis der Restriktionsstellenhäufigkeit mithilfe der in Anspruch 20 angegebenen Formel, um eine gewünschte Anteilsgröße zu erzeugen,
> (xiii) das Herstellen einer Nucleinsäurebibliothek gemäß den Schritten (i) bis (vi) unter Einsatz zumindest eines dieser weiteren Restriktionsenzyme,
> (xiv) gegebenenfalls das Wiederholen der Schritte (vii) bis (xiii), bis die gewünschte Bibliothekengröße erreicht ist.

**22.** Verfahren nach einem der vorangegangenen Ansprüche, worin die Proben-Nucleinsäure Nucleinsäure aus zwei oder mehr unterschiedlichen Quellen umfasst, die gepoolt werden, um eine Bibliothek herzustellen, die Fragmente von jeder davon umfasst.

23. Verfahren zur Identifikation einer begrenzten Population von Markern in einer Proben-Nucleinsäure, wobei das Verfahren Folgendes umfasst:

   (a) das Bereitstellen von Proben-Nucleinsäure aus zumindest zwei unterschiedliche Quellen,
   (b) das Bereitstellen einer Bibliothek, die einen repräsentativen Anteil der Proben-Nucleinsäure enthält, gemäß einem der Ansprüche 1 bis 22,
   (c) das Identifizieren von Unterschieden innerhalb entsprechender Sequenzen aus unterschiedlichen Quellen, die in der Bibliothek enthalten sind.

24. Verfahren nach einem der Ansprüche 1 bis 23 zur Erzeugung einer Nucleinsäurebibliothek, wobei die Bibliothek eine Vielzahl an unterschiedlichen Nucleinsäurefragmenten umfasst, wobei die Kombination dieser Fragmente einen repräsentativen, Anteil der Gesamtheit einer Proben-Nücleinsäure darstellen, worin dem Verfahren folgende Schritte vorangehen:

   (a) das Bereitstellen eines Digitalcomputers, wobei der Computer einen Prozessor, ein Datenspeichersystem, zumindest eine Eingabevorrichtung und zumindest eine Ausgabevorrichtung umfasst,
   (b) das Ausführen zumindest eines Programmcodes auf dem Computer, der auf Basis der Eingabe von einem oder beiden von Folgenden arbeitet:

      (i) einer Bezugssequenz oder Restriktionskarte der Proben-Nucleinsäure,
      (ii) einer Präferenz bezüglich der Anteilsgröße und gegebenenfalls bevorzugten Regionen der Probe, die im Anteil enthalten sein sollen,

   worin der Programmcode eine Funktion in Übereinstimmung mit der folgenden Formel ausführt, sodass eine Ausgabe der Anzahl und der Art der in Schritt (i) des Verfahrens verwendeten unterschiedlichen Restriktionsenzyme und der in Schritt (iii) des Verfahrens ausgewählten Untergruppe von Sätzen bereitgestellt wird, um eine gewünschte Bibliothekengröße zu erzeugen:

$$N_{x1\sim x2} = GP_i^2 \sum_{k=x1}^{k=x2} \prod_{i=1}^{i} (1-P_i)^k$$

   worin Nx1~x2 die Anzahl an Fragmenten mit einer Länge zwischen x1 und x2 ist,
   k die Fragmentlänge ist,
   x1 und x2 die Ober- und Untergrenze des Größenbereichs der Fragmente in der Bibliothek sind,
   Pi die Wahrscheinlichkeit ist, dass an einer vorgegebenen Base eine Restriktionsstelle für das ‚i'-te Enzym vorliegt, und
   G die Größe der Probe ist.

25. Computerprogramm zur Auswahl der Anzahl und der Art der in Schritt (i) eines Verfahrens nach einem der Ansprüche 1 bis 23 verwendeten unterschiedlichen Restriktionsenzyme und der in Schritt (iii) ausgewählten Untergruppe von Sätzen, wobei der Computerprogrammcode auf Basis der Eingabe von einem oder beiden von Folgenden arbeitet:

   (i) einer Bezugssequenz oder Restriktionskarte der Proben-Nucleinsäure,
   (ii) einer Präferenz bezüglich der Anteilsgröße und gegebenenfalls bevorzugten Regionen der Probe, die im Anteil enthalten sein sollen,
   und worin der Programmcode eine Vergleichstabelle umfasst, die Bezugsrestriktionsstellen-Zielsequenzen für unterschiedliche 4- und 6-nt-spaltende Restriktionsenzyme enthält,
   und worin der Programmcode eine Funktion in Übereinstimmung mit der folgenden Formel ausführt, sodass eine Ausgabe der Anzahl und der Art der in Schritt (i) des Verfahrens verwendeten unterschiedlichen Restriktionsenzyme und der in Schritt (iii) des Verfahrens ausgewählten Untergruppe von Sätzen bereitgestellt wird, um eine gewünschte Bibliothekengröße zu erzeugen:

$$N_{x1\sim x2} = GP_1^2 \sum_{k=x1}^{k=x2} \prod_{i=1}^{i}(1-P_i)^k$$

worin Nx1~x2 die Anzahl an Fragmenten mit einer Länge zwischen x1 und x2 ist, k die Fragmentlänge ist, x1 und x2 die Ober- und Untergrenze des Größenbereichs der Fragmente in der Bibliothek sind, Pi die Wahrscheinlichkeit ist, dass an einer vorgegebenen Base eine Restriktionsstelle für das ‚i'-te Enzym vorliegt, und G die Größe der Probe ist.

26. Computerprogramm nach Anspruch 25, das auf einem Speichermedium oder auf einer Vorrichtung gespeichert ist, die von einem programmierbaren Computer für allgemeine oder spezielle Zwecke gelesen werden können.

27. System zur Auswahl der Anzahl und der Art der in Schritt (i) eines Verfahrens nach einem der Ansprüche 1 bis 23 verwendeten unterschiedlichen Restriktionsenzyme und der in Schritt (iii) ausgewählten Untergruppe von Sätzen, wobei das System einen Programmcode umfasst, der auf einem Digitalcomputer läuft, wobei es sich um einen Programmcode nach Anspruch 25 oder Anspruch 26 handelt, und wobei der Computer einen Prozessor, ein Datenspeichersystem, zumindest eine Eingabevorrichtung und zumindest eine Ausgabevorrichtung umfasst.

## Revendications

1. Procédé pour produire une banque d'acide nucléique, laquelle banque contient une pluralité de fragments différents d'acide nucléique, la combinaison desdits fragments étant une partition représentative de la totalité d'un échantillon d'acide nucléique, le procédé comprenant:

   (i) la digestion de l'échantillon d'acide nucléique avec une pluralité de différentes enzymes de restriction pour générer une pluralité de différentes couches de fragments, chaque couche étant un groupe de fragments ayant une combinaison unique d'extrémités de restriction, et la combinaison des couches représentant la totalité de l'échantillon d'acide nucléique,
   (ii) la purification facultative desdits fragments,
   (iii) la sélection d'un sous-ensemble désiré de couches selon les extrémités de restriction uniques desdites couches,
   (iv) la ligature dudit sous-ensemble de couches dans des vecteurs adaptés pour le recevoir,
   (v) la transformation de cellules hôtes avec les vecteurs,
   (vi) la culture desdites cellules hôtes pour fournir ladite banque contenant ladite partition de l'échantillon d'acide nucléique, et la pluralité de différentes enzymes de restriction comprenant (a) une ou deux enzymes qui sont utilisées comme générateurs d'extrémité de clonage pour créer les extrémités de clonage sélectionnées à l'étape (iii) et (b) des enzymes reconnaissant un site à quatre bases qui détruisent tout ou partie des fragments qui pourraient sinon être clonés dans les vecteurs de l'étape (iv).

2. Procédé selon la revendication 1 dans lequel l'échantillon est de l'ADN génomique.

3. Procédé selon l'une quelconque des revendications précédentes dans lequel le nombre et le type des différentes enzymes de restriction utilisées à l'étape (i) et le sous-ensemble de couches sélectionnées à l'étape (iii) sont sélectionnés afin de générer une taille de banque de complexité réduite comparativement à l'échantillon d'acide nucléique d'au moins 10, 100 ou 1 000 fois.

4. Procédé selon l'une quelconque des revendications précédentes dans lequel entre 3 et 6 enzymes de restriction sont utilisées.

5. Procédé selon l'une quelconque des revendications précédentes dans lequel la digestion par une enzyme de restriction est partielle et le groupe de fragments dans la couche sélectionnée ont des extrémités de restriction créées par ladite digestion partielle.

6. Procédé selon l'une quelconque des revendications précédentes dans lequel le sous-ensemble sélectionné de couches consiste en une couche ou deux couches.

7. Procédé selon l'une quelconque des revendications précédentes dans lequel les fragments sont purifiés à l'étape (ii).

8. Procédé selon l'une quelconque des revendications précédentes dans lequel la plage de tailles des fragments dans la banque est entre 100 et 2 000 pb.

9. Procédé selon l'une quelconque des revendications précédentes dans lequel des lieurs sont ajoutés avant ou pendant l'étape (iv) pour empêcher que des sous-ensembles non désirés de couches soient inclus dans ladite banque,
chacun desdits lieurs comprenant:

  (i) une séquence de coeur,
  (ii) une partie qui correspond à l'extrémité restreinte d'un sous-ensemble non désiré, et
  (iii) une séquence pour empêcher les fragments de se recombiner dans le sous-ensemble non désiré.

10. Procédé selon la revendication 9, dans lequel les lieurs comprennent l'un quelconque de ceux qui sont présentés dans le tableau 1.

11. Procédé selon l'une quelconque des revendications précédentes dans lequel des oligonucléotides adaptateurs sont utilisés à l'étape (iv) pour faciliter la ligature du sous-ensemble désiré de couches dans des vecteurs adaptés pour le recevoir.

12. Procédé selon l'une quelconque des revendications précédentes dans lequel des banques sont préparées séparément en utilisant des enzymes de restriction sensibles et non sensibles à la méthylation, la comparaison des banques permettant ainsi de révéler des motifs de distribution de méthylation dans l'échantillon.

13. Procédé selon l'une quelconque des revendications précédentes, dans lequel la séquence de l'échantillon d'acide nucléique n'est pas connue, et le nombre et le type des différentes enzymes de restriction utilisées à l'étape (i), et le sous-ensemble de couches sélectionné à l'étape (iii) sont sélectionnés pour produire la taille de banque désirée conformément à la fréquence de site de restriction de chaque enzyme de la séquence de l'acide nucléique d'échantillon, et sont sélectionnés conformément à la formule suivante:

$$N_{x1-x2} = GP_1^2 \sum_{k=x1}^{k=x2} \prod_{i=1}^{i} (1 - P_i)^k$$

Nx1-x2 est le nombre de fragments ayant une longueur entre x1 et x2
k est la longueur du fragment
x1 et x2 sont les limites supérieure et inférieure de la plage de tailles des fragments dans la banque
Pi est la probabilité qu'il y ait un site de restriction au niveau d'une base donnée quelconque pour l'enzyme n° « i »
G est la taille de l'échantillon.

14. Procédé selon l'une quelconque des revendications 1 à 12 dans lequel la taille de l'échantillon d'acide nucléique est connue, et le nombre et le type des différentes enzymes de restriction utilisées à l'étape (i), et le sous-ensemble de couches sélectionnées à l'étape (iii) sont sélectionnés pour produire la taille de banque désirée conformément à une fréquence supposée de sites de restriction de chaque enzyme dans l'échantillon d'acide nucléique, et sont sélectionnés conformément à la formule suivante:

$$N_{x1-x2} = GP_1^2 \sum_{k=x1}^{k=x2} \prod_{i=1}^{i} (1 - P_i)^k$$

Nx1-x2 est le nombre de fragments ayant une longueur entre x1 et x2

G est la taille de l'échantillon.

x1 et x2 sont les limites supérieure et inférieure de la plage de tailles des fragments dans la banque

Pi est la probabilité qu'il y ait un site de restriction au niveau d'une base donnée quelconque pour l'enzyme n° « i ».

**15.** Procédé selon la revendication 14 dans lequel les enzymes de restriction utilisées à l'étape (i) sont des enzymes de restriction reconnaissant des sites à quatre nucléotides et à 6 nucléotides, et sont choisies en se fondant sur la formule suivante:

$$N' = 4^{-12} v' G \sum_{k=x1}^{k=x2} \left[ (1 - 1/4^4)^{nk} (1 - 1/4^6)^{(1+m)k} \right]$$

dans laquelle:

k est la longueur du fragment

G est la taille de l'échantillon

x1 et x2 sont les limites supérieure et inférieure de la plage de tailles des fragments dans la banque

n est le nombre d'enzymes supplémentaires reconnaissant des sites à 4 nucléotides

m est le nombre d'enzymes supplémentaires reconnaissant des sites à 6 nucléotides.

**16.** Procédé selon les revendications 14 ou 15, dans lequel la taille de la banque obtenue est estimée par les étapes suivantes consistant à:

(vii) séquencer les fragments dans une fraction des cellules hôtes dans ladite banque;

(viii) estimer la taille de la banque en utilisant la formule suivante:

$$F = n(n-1) / \sum_i n_i(n_i - 1) \pm s$$

dans laquelle:

F est la taille estimée de la banque

n est le nombre total de séquences obtenues par séquençage,

ni est le nombre de séquences dans le contig N° i

s est l'erreur standard.

**17.** Procédé selon la revendication 16, dans lequel une banque est générée par les étapes supplémentaires consistant à:

(ix) fournir une fréquence de site de restriction pour des enzymes non utilisées à l'étape (i), en utilisant facultativement les informations de séquences obtenues à l'étape (vii),

(x) sélectionner d'autres enzymes de restriction en se fondant sur la fréquence de site de restriction pour générer une taille désirée de partition en utilisant la formule donnée à la revendication 16,

(xi) produire une banque nucléique conformément aux étapes (i)-(vi) en utilisant au moins l'une de ces enzymes de restriction supplémentaires,

(xii) répéter facultativement les étapes (vii) à (xi) jusqu'à ce que la taille de banque désirée soit obtenue.

**18.** Procédé selon l'une quelconque des revendications 1 à 12 dans lequel la taille de l'échantillon d'acide nucléique n'est pas connue,

et le nombre et le type des différentes enzymes de restriction utilisées à l'étape (i) et le sous-ensemble de couches sélectionnées à l'étape (iii) sont sélectionnés de manière à produire la taille de banque désirée conformément à une fréquence supposée de site de restriction de chaque enzyme dans l'échantillon d'acide nucléique et dans lequel la taille de la banque obtenue est estimée par les étapes supplémentaires consistant à:

(vii) séquencer les fragments dans une fraction des cellules hôtes dans ladite banque;
(viii) estimer la taille de la banque en utilisant la formule suivante:

$$F = n(n-1) / \sum_i n_i (n_i - 1) \pm s$$

dans laquelle:

F est la taille estimée de la banque
n est le nombre total de séquences obtenues par séquençage,
ni est le nombre de séquences dans le contig N° i
s est l'erreur standard.

19. Procédé selon la revendication 18 dans lequel trois enzymes reconnaissant des sites de 4 nucléotides et une enzyme reconnaissant des sites de six nucléotides sont utilisées à l'étape (i).

20. Procédé selon la revendication 18 ou la revendication 19, dans lequel la taille de l'échantillon est estimée par les étapes supplémentaires consistant à:

(ix) fournir la fréquence de site de restriction des enzymes utilisées à l'étape (i), en utilisant facultativement les informations de séquence obtenues à l'étape (vii),
(x) calculer la taille G de l'échantillon en utilisant la formule suivante:

$$N_{x1-x2} = GP_1^2 \sum_{k=x1}^{k=x2} \prod_{l=1}^{j} (1 - P_l)^k$$

dans laquelle:

Nx1-x2 est le nombre de fragments ayant une longueur entre x1 et x2
k est la longueur du fragment
x1 et x2 sont les limites supérieure et inférieure de la plage de tailles des fragments dans la banque
Pi est la probabilité qu'il y ait un site de restriction au niveau d'une base donnée quelconque pour l'enzyme n° « i »
G est la taille de l'échantillon.

21. Procédé selon la revendication 20, dans lequel une banque est générée par les étapes supplémentaires consistant à:

(xi) fournir une fréquence de site de restriction pour des enzymes non utilisées à l'étape (i), facultativement en utilisant les informations de séquence obtenues à l'étape (vii),
(xii) sélectionner d'autres enzymes de restriction en se fondant sur la fréquence de site de restriction pour générer une taille désirée de partition en utilisant la formule donnée à la revendication 20,
(xiii) produire une banque nucléique conformément aux étapes (i) - (vi) en utilisant au moins l'une de ces enzymes de restriction supplémentaires,
(xiv) répéter facultativement les étapes (vii) à (xiii) jusqu'à ce que la taille de banque désirée soit obtenue.

22. Procédé selon l'une quelconque des revendications précédentes dans lequel l'échantillon d'acide nucléique comprend l'acide nucléique de deux sources différentes ou plus qui sont regroupés pour produire une banque comprenant des fragments de chaque.

23. Procédé pour identifier une population limitée de marqueurs dans un échantillon d'acide nucléique, lequel procédé comprend:

(a) la fourniture d'un échantillon d'acide nucléique provenant d'au moins deux sources différentes,
(b) la fourniture d'une banque contenant une partition représentative de l'échantillon d'acide nucléique conformément à l'une quelconque des revendications 1 à 22,
(c) l'identification de différences dans des séquences correspondantes issues desdites sources différentes contenues dans la banque.

24. Procédé selon l'une quelconque des revendications 1 à 23 pour produire une banque d'acide nucléique, laquelle banque contient une pluralité de différents fragments d'acide nucléique, la combinaison desdits fragments étant une partition représentative de la totalité d'un échantillon d'acide nucléique,
dans lequel ledit procédé est précédé par les étapes consistant à:

(a) fournir un ordinateur numérique, lequel ordinateur comprend un processeur, un système de mémorisation de données, au moins un dispositif d'entrée et au moins un dispositif de sortie,
(b) exécuter un code de logiciel sur ledit ordinateur, qui fonctionne sur l'entrée de:

(I) une séquence de référence ou carte de restriction issues de l'échantillon d'acide nucléique et/ou
(II) une préférence concernant la taille de partition, et facultativement de région préférée de l'échantillon à inclure dans la partition,

dans lequel le code de logiciel remplit une fonction conformément à la formule suivante de manière à fournir une sortie du nombre et du type des différentes enzymes de restriction à utiliser à l'étape (i) du procédé, et du sous-ensemble de couches à sélectionner à l'étape (iii) du procédé, de manière à produire une taille de banque désirée:

$$N_{x1-x2} = GP_1^2 \sum_{k=x1}^{k=x2} \prod_{i=1}^{l} (1-P_i)^k$$

Nx1-x2 est le nombre de fragments ayant une longueur entre x1 et x2
k est la longueur du fragment
x1 et x2 sont les limites supérieure et inférieure de la plage de tailles des fragments dans la banque
Pi est la probabilité qu'il y ait un site de restriction au niveau d'une base donnée quelconque pour l'enzyme n° « i »
G est la taille de l'échantillon.

25. Logiciel pour sélectionner le nombre et le type des différentes enzymes de restriction utilisées à l'étape (i), et le sous-ensemble de couches sélectionné à l'étape (iii) du procédé selon l'une quelconque des revendications 1 à 23, lequel code de logiciel fonctionne sur l'entrée de:

(i) une séquence de référence ou carte de restriction issues de l'échantillon d'acide nucléique et/ou
(ii) une préférence concernant la taille de partition, et facultativement de région préférée de l'échantillon à inclure dans la partition,
et dans lequel le code de logiciel inclut une table de vérification incluant des séquences cibles de site de restriction pour différentes enzymes reconnaissant des sites de 4 nucléotides et de 6 nucléotides,
et dans lequel le code de programme remplit une fonction conformément à la formule suivante de manière à fournir une sortie du nombre et du type des différentes enzymes de restriction à utiliser à l'étape (i) du procédé, et du sous-ensemble de couches à sélectionner à l'étape (iii) du procédé, de manière à produire une taille de banque désirée:

$$N_{x1-x2} = GP_1^2 \sum_{k=x1}^{k=x2} \prod_{i=1}^{l} (1-P_i)^k$$

Nx1-x2 est le nombre de fragments ayant une longueur entre x1 et x2
k est la longueur du fragment

x1 et x2 sont les limites supérieure et inférieure de la plage de tailles des fragments dans la banque

Pi est la probabilité qu'il y ait un site de restriction au niveau d'une base donnée quelconque pour l'enzyme n° « i »

G est la taille de l'échantillon.

26. Logiciel selon la revendication 25, qui est enregistré sur un support d'enregistrement ou un dispositif d'enregistrement lisible par un ordinateur programmable généraliste ou spécialisé.

27. Système pour sélectionner le nombre et le type des différentes enzymes de restriction utilisées à l'étape (i), et le sous-ensemble de couches sélectionné à l'étape (iii) du procédé selon l'une quelconque des revendications 1 à 23, lequel système comprend un code de logiciel exécuté sur un ordinateur numérique, qui est le code de logiciel de la revendication 25 ou de la revendication 26,
et lequel ordinateur comprend un processeur, un système de mémorisation de données, au moins un dispositif d'entrée et au moins un dispositif de sortie.